# EUROPEAN PATENT APPLICATION

(11) **EP 4 393 906 A1**
(43) Date of publication of application: **03.07.2024**
(21) Application number: 22860616.6
(22) Date of filing: 26.08.2022
(51) Int. Cl.: C07D 209/08, C07D 307/79, A61K 31/404, A61K 31/343, A61P 35/00

(54) **SOLID FORM OF INDOLE COMPOUND, PREPARATION METHOD THEREFOR AND USE THEREOF**

(30) Priority: 27.08.2021 CN 202111000027
(71) Applicant: Keythera (Suzhou) Bio-Pharmaceuticals Co., Limited, Suzhou, Jiangsu 215000 (CN)
(72) Inventor: DENG, Yongqi, Suzhou, Jiangsu 215000 (CN); TIAN, Yuan, Suzhou, Jiangsu 215000 (CN)
(74) Representative: Maikowski & Ninnemann Patentanwälte Partnerschaft mbB
(86) International application number: PCT/CN2022/115010
(87) International publication number: WO 2023/025270

(57) **Abstract**

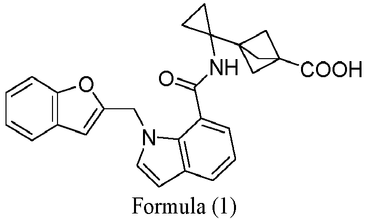

The present application relates to a solid form of an indole compound, a preparation method therefor and a use thereof. Specifically, the present application relates to a solid form of a compound of Formula (1), a method of preparing same, a pharmaceutical composition comprising same, and a use thereof in treating a disease.

## Description

### FIELD OF THE INVENTION

The present application relates to a solid form of an indole compound, a preparation method therefor and a use thereof. Specifically, the present application relates to a solid form of 3-(1-(1-(benzofuran-2-ylmethyl)-1H-indole-7-carboxamido)cyclopropyl)bicyclo[1.1.1]pentane-1-carboxyfi c acid (hereinafter referred to as "a compound of Formula (1)"), a method of preparing the same, a pharmaceutical composition comprising the same, and a use thereof in treating a disease.

### BACKGROUND OF THE INVENTION

Prostaglandin E2 (PGE2) is a derivative of arachidonic acid that can inhibit the function of immune cells and evade anti-tumor immunity. PGE2 regulates biological functions through 4 types of PGE2 receptors (EP1, EP2, EP3, and EP4). EP4 is the primary PGE2 receptor in tumor tissues and is involved in PGE2-promoted tumor progression. There is evidence that the expression of the EP4 receptor is increased in many types of tumor tissues. A large body of evidence also suggests that the levels of PGE2 are elevated in many tumor tissues and that through the EP4 receptor, the function of immune cells in tumor tissues is suppressed, allowing tumor cells to escape the anti-tumor immune system, thereby accelerating tumor growth and metastasis. EP4 receptor antagonists can block these effects of PGE2, thereby enhancing anti-tumor immune function.

Furthermore, scientific evidence suggests that selective EP4 antagonists may be an effective medication for relieving inflammatory pain, with better gastrointestinal tolerance than current standard anti-inflammatory analgesic drugs such as NSAIDs and COX-2 inhibitors. Notably, since EP4 antagonists do not directly interfere with the biosynthesis of prostaglandin E (PGE2) and other prostaglandins (such as prostacyclin and thromboxane), these drugs may have better cardiovascular safety.

Given the potential applications of EP4 antagonists in tumor immunity and anti-inflammatory analgesia, several EP4 antagonists are in the clinical research stage. However, as of now, no such drugs have been approved for marketing. There are also few reports on the solid form of this class of drugs.

### SUMMARY OF THE INVENTION

An aspect of the present application provides crystalline forms of the compound of Formula (1) (3-(1-(1-(benzofuran-2-ylmethyl)-1H-indole-7-carboxamido)cyclopropyl)bicyclo[1.1.1]pentane-1-carboxyl ic acid, which is a compound that inhibits PGE2/EP4 signaling pathway) as shown below:

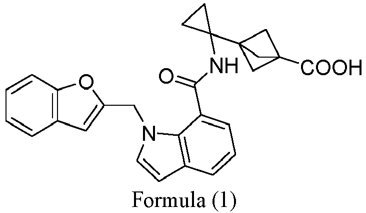

The preferred crystalline form of the compound of Formula (1) of the present application possesses excellent physical properties (including solubility, dissolution rate, low hygroscopicity, high-temperature resistance, high humidity resistance, flowability, *etc*.), and in terms of properties such as bioavailability, physical and/or chemical stability, and ease of preparation, the preferred crystalline form of the present invention can exhibit superior characteristics. The preferred crystalline form of the present application exhibits good powder flow properties, making it more suitable and convenient for mass production and formulation, effectively ensuring the quality and efficacy of the pharmaceutical product.

The preferred crystalline form of the compound of Formula (1) of the present application demonstrates good chemical and thermal stability, thereby facilitating complete dissolution during administration and formulation, and maintaining sufficient biological activity. Additionally, the preferred crystalline form of the compound of Formula (1) of the present application exhibits high bioavailability, providing an effective therapeutic dose of the compound of Formula (1) *in vivo.*

By grinding the preferred crystalline form of the compound of Formula (1) of the present application to produce a fine powder, and then subjecting said fine powder to X-ray powder diffraction (XRPD) analysis, experimental results indicate that there is no change in crystalline form. This demonstrates that the preferred crystalline form of the present application has good stability, is easy to prepare, and is more suitable for the preparation of formulations.

The preferred crystalline form of the compound of Formula (1) of the present application possesses good flowability and particle shape, as well as significantly improved viscosity, which can greatly reduce filtration time during the formulation process, shorten the production cycle, and save costs.

Another aspect of the present application provides a method of preparing the crystalline form of the application, wherein the method includes, but is not limited to, a room temperature solvent volatilization method, a suspension stirring method, an anti-solvent addition method and a cooling method.

Another aspect of the present application provides a pharmaceutical composition comprising one or more crystalline forms of the present application, and one or more pharmaceutically acceptable carriers.

Another aspect of the present application provides use of the crystalline form of the present application in the manufacture of a medicament for treating acute or chronic pain, migraine, osteoarthritis, rheumatoid arthritis, gout, bursitis, ankylosing spondylitis, primary dysmenorrhea, cancer or arteriosclerosis.

### BRIEF DESCRIPTION OF THE DRAWINGS

Figure 1 is the XRPD pattern of crystalline form I of the compound of Formula (1).
Figure 2 is the DSC and TGA graph of crystalline form I of the compound of Formula (1).
Figure 3 is the XRPD pattern of crystalline form II of the compound of Formula (1).
Figure 4 is the DSC and TGA graph of crystalline form II of the compound of Formula (1).
Figure 5 is the XRPD pattern of crystalline form III of the compound of Formula (1).
Figure 6 is the DSC and TGA graph of crystalline form III of the compound of Formula (1).
Figure 7 is the XRPD pattern of crystalline form IV of the compound of Formula (1).
Figure 8 is the DSC and TGA graph of crystalline form IV of the compound of Formula (1).
Figure 9 is the XRPD pattern of crystalline form V of the compound of Formula (1).
Figure 10 is the DSC and TGA graph of crystalline form V of the compound of Formula (1).
Figure 11 is the XRPD pattern comparison of the sample before and after the grinding test in Experimental Example 1.
Figure 12 is the XRPD pattern comparison of crystalline form IV before and after heating in Experimental Example 2.
Figure 13 is the XRPD pattern comparison of crystalline form II before and after the test in Experimental Example 3.

### DETAILED DESCRIPTION OF THE INVENTION

### DEFINITIONS

Unless otherwise defined in the context, all technical and scientific terms used herein are intended to have the same meaning as commonly understood by a person skilled in the art. References to techniques employed herein are intended to refer to the techniques as commonly understood in the art, including variations on those techniques or substitutions of equivalent techniques which would be apparent to a person skilled in the art. While it is believed that most of the following terms will be readily understood by a person skilled in the art, the following definitions are nevertheless put forth to better illustrate the present invention.

The terms "contain", "include", "comprise", "have", or "relate to", as well as other variations used herein are inclusive or open-ended, and do not exclude additional, unrecited elements or method steps.

The word "about" as used herein refers to, as appreciated by a person skilled in the art, a range within the acceptable standard error of a value, such as ±0.05, ±0.1, ±0.2, ±0.3, ±1, ±2 or ±3, *etc.*

The term "solid form" as used herein includes all solid forms of the compounds of Formula (1), such as a crystalline form or amorphous form.

The term "amorphous" as used herein refers to any solid substance which lacks order in three dimensions. In some instances, amorphous solids may be characterized by known techniques, including XRPD crystallography, solid state nuclear magnet resonance (ssNMR) spectroscopy, DSC, or some combination of these techniques. As illustrated below, amorphous solids give diffuse XRPD patterns, typically comprised of one or two broad peaks (i.e., peaks having base widths of about 5° 2θ or greater).

The term "crystalline form" or "crystal" as used herein refers to any solid substance exhibiting three-dimensional order, which in contrast to an amorphous solid substance, gives a distinctive XRPD pattern with sharply defined peaks.

The term "X-ray powder diffraction pattern (XRPD pattern)" as used herein refers to the experimentally observed diffractogram or parameters derived therefrom. XRPD patterns are usually characterized by peak positions (abscissa) and peak intensities (ordinate). The XRPD pattern in the present invention is preferably collected on PANalytacal Empyrean and X'Pert3 X-ray powder diffractometer, and the transmissive mode is preferably collected on PANalytacal Empyrean X-ray powder diffractometer.

The term "2θ" as used herein refers to the peak position in degrees based on the experimental setup of the X-ray diffraction experiment and is a common abscissa unit in diffraction patterns. The experimental setup requires that if a reflection is diffracted when the incoming beam forms an angle theta (θ) with a certain lattice plane, the reflected beam is recorded at an angle 2 theta (2Θ). It should be understood that reference herein to specific 2θ values for a specific solid form is intended to mean the 2θ values (in degrees) as measured using the X-ray diffraction experimental conditions as described herein. For example, as described herein, Cu-Kα (Kα1 (Å): 1.540598 and Kα2 (Å): 1.544426 Å) was used as the source of radiation.

The term "differential scanning calorimetry (DSC) graph" as used herein refers to a curve recorded on a differential scanning calorimeter. The DSC graph in the present application is preferably collected on a Discovery DSC 250 (TA Instruments, US).

As used herein, the term "essentially the same" with reference to X-ray diffraction peak positions means that typical peak position and intensity variability are taken into account. For example, one skilled in the art will appreciate that the peak positions (2Θ) will show some variability, typically as much as 0.1 to 0.2 degree, as well as on the apparatus being used to measure the diffraction. Further, one skilled in the art will appreciate that relative peak intensities will show inter-apparatus variability as well as variability due to degree of crystallinity, preferred orientation, prepared sample surface, and other factors known to those skilled in the art, and should be taken as qualitative measures only. Similarly, as used herein, "essentially the same" with reference to the DSC graph is intended to also encompass the variabilities associated with these analytical techniques, which are known to those of skill in the art. For example, a differential scanning calorimetry graph will typically have a variability of up to ±0.2°C for well defined peaks, and even larger for broad lines (e.g., up to ±1°C).

The liquid nuclear magnetic resonance spectrum in the present application is preferably collected on a Bruker 400M nuclear magnetic resonance spectrometer, with DMSO-*d₆* as the solvent, unless otherwise stated.

The term "hydrocarbons" as used herein preferably means hydrocarbons having 1 to 10 carbon atoms, including alkanes, halogenated alkanes, alkenes, alkynes, and aromatic hydrocarbons, specifically including, but not limited to, dichloromethane, trichloromethane (chloroform), n-hexane, n-heptane and toluene.

The term "alcohols" as used herein preferably means alcohols having 1 to 10 carbon atoms, including, but not limited to, methanol, ethanol, 1-propanol (n-propanol), 2-propanol (isopropanol), 1-butanol, 2-butanol and *tert*-butanol.

The term "ethers" as used herein preferably means ethers having 2 to 6 carbon atoms, including chain ethers and cyclic ethers (e.g., furans (including tetrahydrofurans) and dioxanes), specifically including, but not limited to, diethyl ether, diisopropyl ether, methyl *tert*-butyl ether, tetrahydrofuran, 2-methyltetrahydrofuran, dioxane, cyclopentyl methyl ether, anisole and dimethoxyethane.

The term "nitriles" as used herein preferably means nitriles having 2 to 6 carbon atoms, including, but not limited to, acetonitrile and propionitrile.

The term "ketones solvent" as used herein preferably means ketones having 2 to 6 carbon atoms, including, but not limited to, acetone, butanone, methyl ethyl ketone, methyl isobutyl ketone, and diethyl ketone.

The term "esters" as used herein preferably means esters having 3 to 10 carbon atoms, including, but not limited to, ethyl acetate, propyl acetate, isopropyl acetate, ethyl isopropionate, dimethyl carbonate and butyl acetate.

The term "organic acids" as used herein preferably means organic acids having 1 to 10 carbon atoms, including, but not limited to, formic acid and acetic acid.

The term "sulfones" as used herein preferably means sulfones or sulfoxides having 2 to 10 carbon atoms, including, but not limited to, dimethyl sulfoxide.

The term "amides" as used herein preferably means amides having 1 to 10 carbon atoms, including, but not limited to, dimethylformamide or dimethylacetamide.

The term "nitrogen-containing heterocycles" as used herein preferably means nitrogen-containing heterocycles having 3 to 10 carbon atoms and at least one nitrogen atom, including, but not limited to, N-methylpyrrolidone.

Numerical ranges (e.g., "1 to 10") and subranges thereof (e.g., "2 to 10", "2 to 6", "3 to 10"), *etc.* as used herein encompass any point within the numerical range (for example, 1, 2, 3, 4, 5, 6, 7, 8, 9, or 10).

The prepared salt or crystalline form thereof may be recovered by methods including decantation, centrifugation, evaporation, gravity filtration, suction filtration, or any other technique for the recovery of solids under pressure or under reduced pressure. The recovered solid may optionally be dried. "Drying" in the present invention is carried out under reduced pressure (preferably in vacuum) until the residual solvent content is lowered within the limits given in the International Conference on Harmonisation of Technical Requirements for Registration of Pharmaceuticals for Human Use ("ICH") guidelines. The residual solvent content depends on the type of the solvent, but does not exceed about 5000 ppm, or preferably about 4000 ppm, or more preferably about 3000 ppm. Drying may be carried out in a tray dryer, vacuum oven, air oven, cone vacuum dryer, rotary vacuum dryer, fluidized bed dryer, spin flash dryer, flash dryer, or the like. The drying may be carried out at temperatures less than about 100°C, less than about 80°C, less than about 60°C, less than about 50°C, less than about 30°C, or any other suitable temperatures, at atmospheric pressure or under a reduced pressure (preferably in vacuum) for any desired period (e.g., about 1, 2, 3, 5, 10, 15, 20, 24 hours or overnight) until the desired result is achieved, as long as the salt is not degraded in quality. The drying can be carried out any desired times until the desired product quality is achieved. The dried product may optionally be subjected to a size reduction procedure to produce desired particle sizes. Milling or micronization may be performed before drying, or after the completion of drying of the product. Techniques that may be used for particle size reduction include, without limitation, ball, roller and hammer milling, and jet milling.

The term "anhydrous crystalline form" as used herein preferably means a crystalline form wherein no water molecule is comprised as a structural element.

### Crystalline form and preparation method thereof

In an embodiment, the present invention provides crystalline form I of the compound of Formula (1), wherein the crystalline form I has an XRPD pattern comprising characteristic peaks at diffraction angles (2Θ) of about 6.3±0.2°, 11.0±0.2°, 13.5±0.2°, 16.7±0.2°, 18.3±0.2°, 18.6±0.2°, 19.0±0.2°, 22.1±0.2°, 22.8±0.2° and 25.3±0.2°.

In a preferred embodiment, the crystalline form I of the compound of Formula (1) has an XRPD pattern comprising peaks at the following diffraction angles (20):

| 2θ (°) ± 0.2° | Intensity% ± 5% |
|---|---|
| 6.3 | 64 |
| 11.0 | 47 |
| 13.5 | 38 |
| 16.7 | 100 |
| 18.3 | 30 |
| 18.6 | 20 |
| 19.0 | 22 |
| 22.1 | 17 |
| 22.8 | 15 |
| 25.3 | 21 |

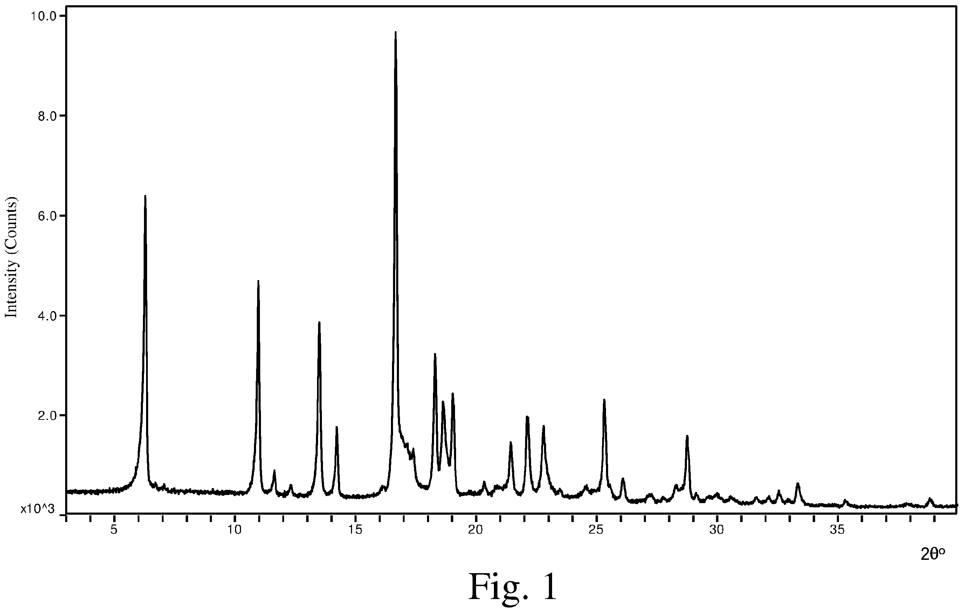

In a more preferred embodiment, the crystalline form I of the compound of Formula (1) has an XRPD pattern comprising peaks at diffraction angles (2Θ) essentially the same as shown in Figure 1. In the most preferred embodiment, the XRPD pattern of the crystalline form I of the compound of Formula (1) is essentially the same as shown in Figure 1, and preferably is as shown in Figure 1.

In a preferred embodiment, the crystalline form I of the compound of Formula (1) of the present invention has a DSC graph comprising a characteristic peak at about 220 ± 2°C (the onset temperature).

In a preferred embodiment, the crystalline form I of the compound of Formula (1) of the present invention has a weight loss of less than about 0.2% when heated to about 200°C.

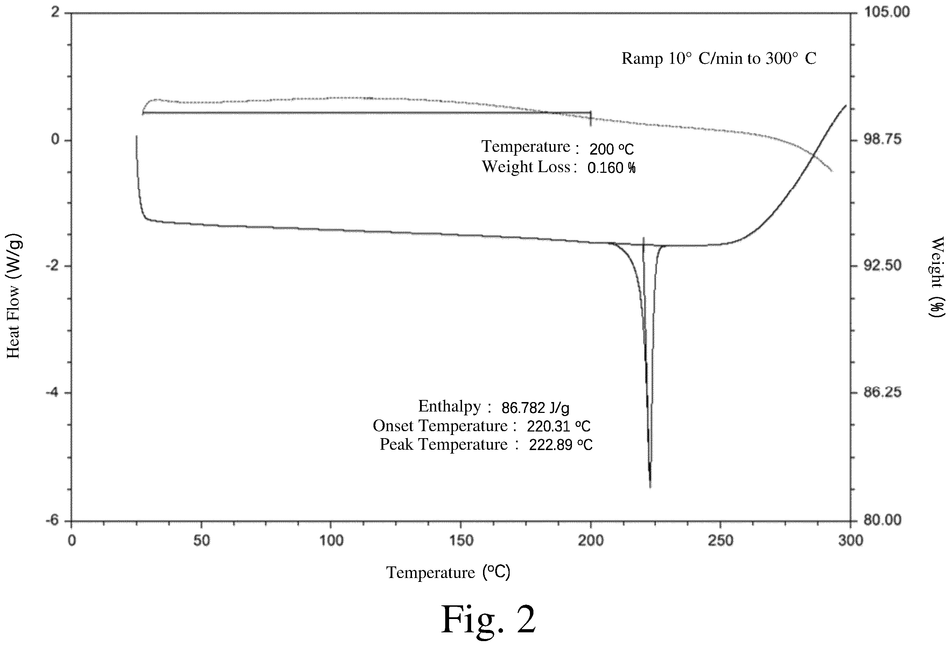

In a more preferred embodiment, the crystalline form I of the compound of Formula (1) has a DSC-TGA graph comprising characteristic peaks essentially the same as shown in Figure 2. In the most preferred embodiment, the DSC-TGA graph of the crystalline form I of the compound of Formula (1) is essentially the same as shown in Figure 2, and preferably is as shown in Figure 2.

In a preferred embodiment, the crystalline form I of the compound of Formula (1) is an anhydrous crystalline form.

In an embodiment, the present invention provides crystalline form II of the compound of Formula (1), wherein the crystalline form II has an XRPD pattern comprising characteristic peaks at diffraction angles (2Θ) of about 6.3±0.2°, 11.1±0.2°, 13.8±0.2°, 16.6±0.2°, 16.8±0.2°, 18.2±0.2°, 19.2±0.2°, 22.4±0.2°, 22.8±0.2° and 25.2±0.2°.

In a preferred embodiment, the crystalline form II of the compound of Formula (1) has an XRPD pattern comprising peaks at the following diffraction angles (2θ):

| 2θ (°) ± 0.2° | Intensity% ± 5% |
|---|---|
| 6.3 | 45 |
| 11.1 | 38 |
| 13.8 | 34 |
| 16.6 | 36 |
| 16.8 | 100 |
| 18.2 | 22 |
| 19.2 | 13 |
| 22.4 | 18 |
| 22.8 | 23 |
| 25.2 | 17 |

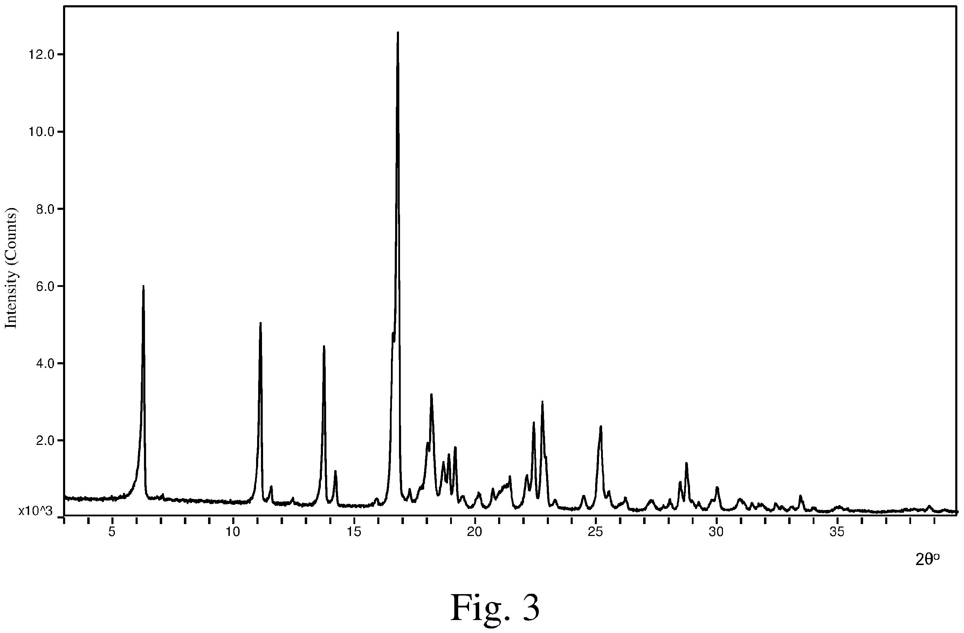

In a more preferred embodiment, the crystalline form II of the compound of Formula (1) has an XRPD pattern comprising peaks at diffraction angles (2Θ) essentially the same as shown in Figure 3. In the most preferred embodiment, the XRPD pattern of the crystalline form II of the compound of Formula (1) is essentially the same as shown in Figure 3, and preferably is as shown in Figure 3.

In a preferred embodiment, the crystalline form II of the compound of Formula (1) of the present invention has a DSC graph comprising a characteristic peak at about 224 ± 2°C (the onset temperature).

In a preferred embodiment, the crystalline form II of the compound of Formula (1) of the present invention has almost no weight loss when heated to about 200°C.

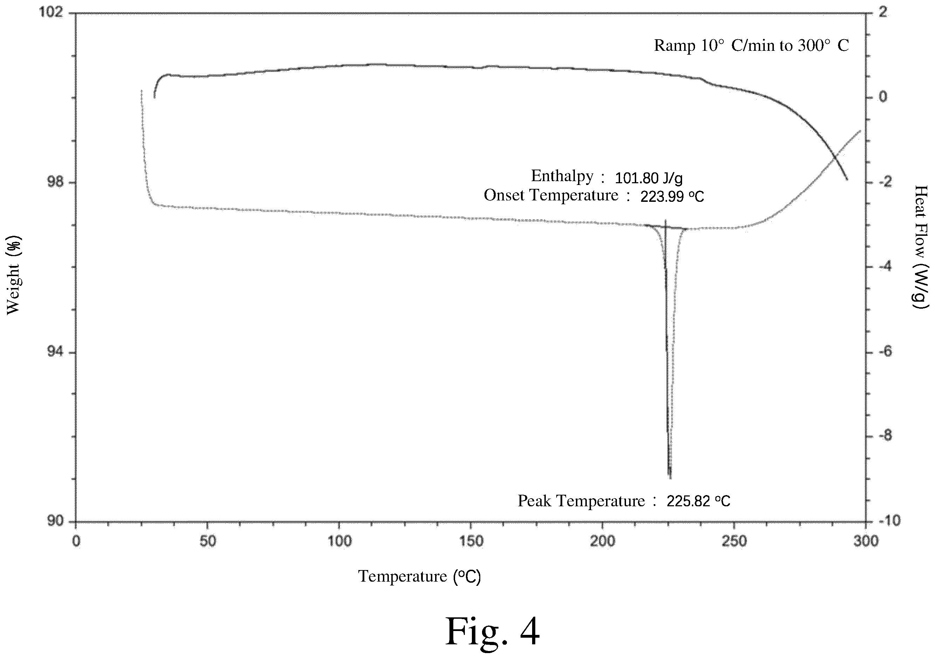

In a more preferred embodiment, the crystalline form II of the compound of Formula (1) has a DSC-TGA graph comprising characteristic peaks essentially the same as shown in Figure 4. In the most preferred embodiment, the DSC-TGA graph of the crystalline form II of the compound of Formula (1) is essentially the same as shown in Figure 4, and preferably is as shown in Figure 4.

In a preferred embodiment, the crystalline form II of the compound of Formula (1) is an anhydrous crystalline form.

In an embodiment, the present invention provides crystalline form III of the compound of Formula (1), wherein the crystalline form III has an XRPD pattern comprising characteristic peaks at diffraction angles (2Θ) of about 5.3±0.2°, 10.7±0.2°, 12.0±0.2°, 17.7±0.2°, 18.1±0.2°, 21.6±0.2°, 22.1±0.2°, 26.9±0.2°, 31.9±0.2° and 32.6±0.2°.

In a preferred embodiment, the crystalline form III of the compound of Formula (1) has an XRPD pattern comprising peaks at the following diffraction angles (20):

| 2θ (°) ± 0.2° | Intensity% ± 5% |
|---|---|
| 5.3 | 64 |
| 10.7 | 59 |
| 12.0 | 7 |
| 17.7 | 8 |
| 18.1 | 60 |
| 21.6 | 100 |
| 22.1 | 36 |
| 26.9 | 10 |
| 31.9 | 8 |
| 32.6 | 7 |

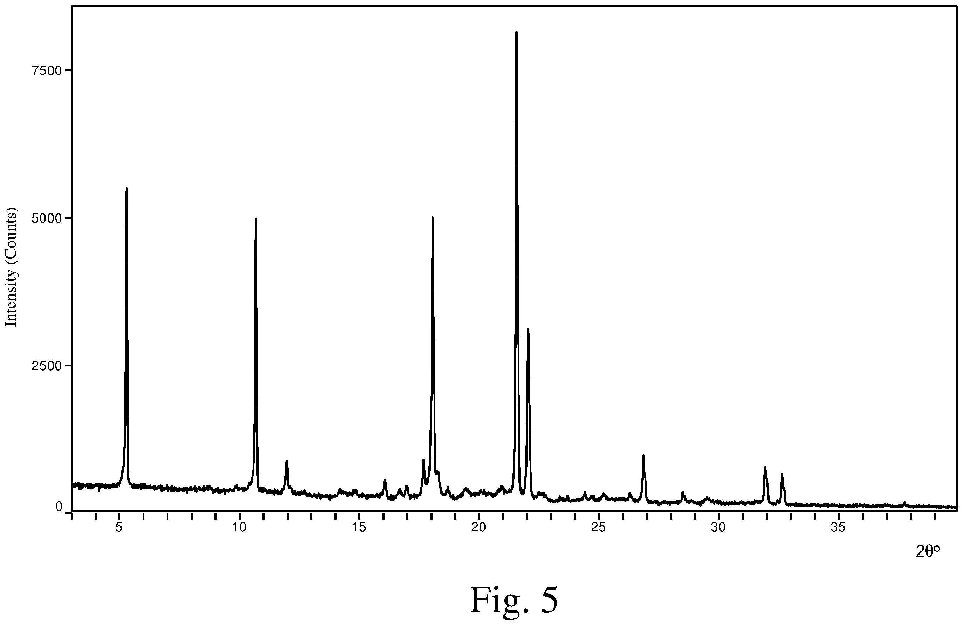

In a more preferred embodiment, the crystalline form III of the compound of Formula (1) has an XRPD pattern comprising peaks at diffraction angles (2Θ) essentially the same as shown in Figure 5. In the most preferred embodiment, the XRPD pattern of the crystalline form III of the compound of Formula (1) is essentially the same as shown in Figure 5, and preferably is as shown in Figure 5.

In a preferred embodiment, the crystalline form III of the compound of Formula (1) of the present invention has a DSC graph comprising characteristic peaks at about 97 ± 2°C and 223 ± 2°C (the onset temperature).

In a preferred embodiment, the crystalline form III of the compound of Formula (1) of the present invention has a weight loss of about 14% when heated to about 125°C.

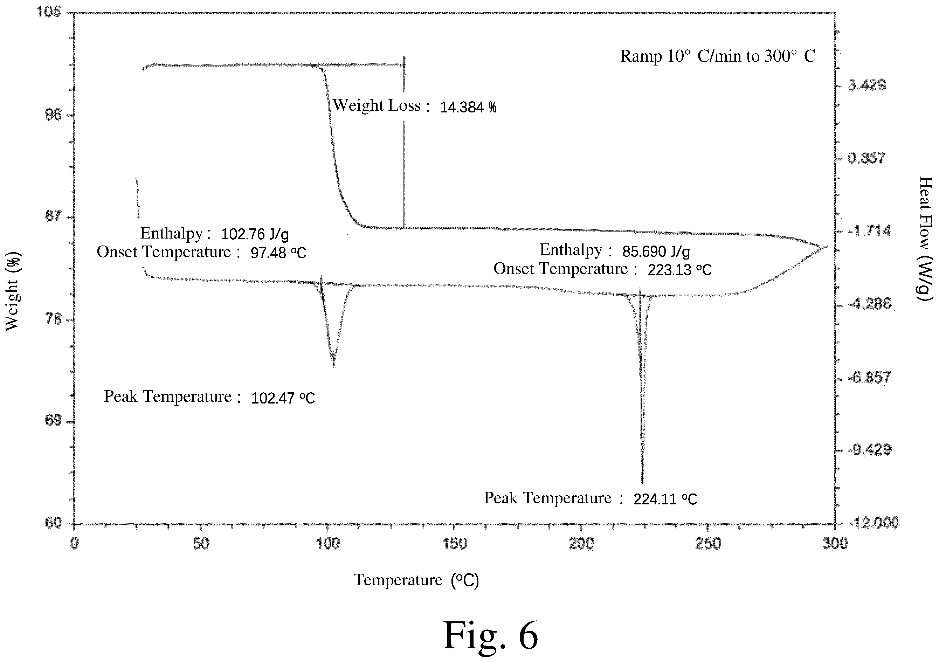

In a more preferred embodiment, the crystalline form III of the compound of Formula (1) has a DSC-TGA graph comprising characteristic peaks essentially the same as shown in Figure 6. In the most preferred embodiment, the DSC-TGA graph of the crystalline form III of the compound of Formula (1) is essentially the same as shown in Figure 6, and preferably is as shown in Figure 6.

In a preferred embodiment, the crystalline form III of the compound of Formula (1) is a solvate with tetrahydrofuran, wherein the molar ratio of the compound of Formula (1) to tetrahydrofuran preferably is 1:1.

In an embodiment, the present invention provides crystalline form IV of the compound of Formula (1), wherein the crystalline form IV has an XRPD pattern comprising characteristic peaks at diffraction angles (2Θ) of about 8.7±0.2°, 12.1±0.2°, 12.7±0.2°, 15.5±0.2°, 18.3±0.2°, 18.8±0.2°, 19.3±0.2°, 19.9±0.2°, 21.5±0.2°, 24.4±0.2° and 27.8±0.2°.

In a preferred embodiment, the crystalline form IV of the compound of Formula (1) has an XRPD pattern comprising peaks at the following diffraction angles (20):

| 2θ (°) ± 0.2° | Intensity% ± 5% |
|---|---|
| 8.7 | 83 |
| 12.1 | 35 |
| 12.7 | 83 |
| 15.5 | 100 |
| 18.3 | 31 |
| 18.8 | 62 |
| 19.3 | 53 |
| 19.9 | 48 |
| 21.5 | 72 |
| 24.4 | 37 |
| 27.8 | 37 |

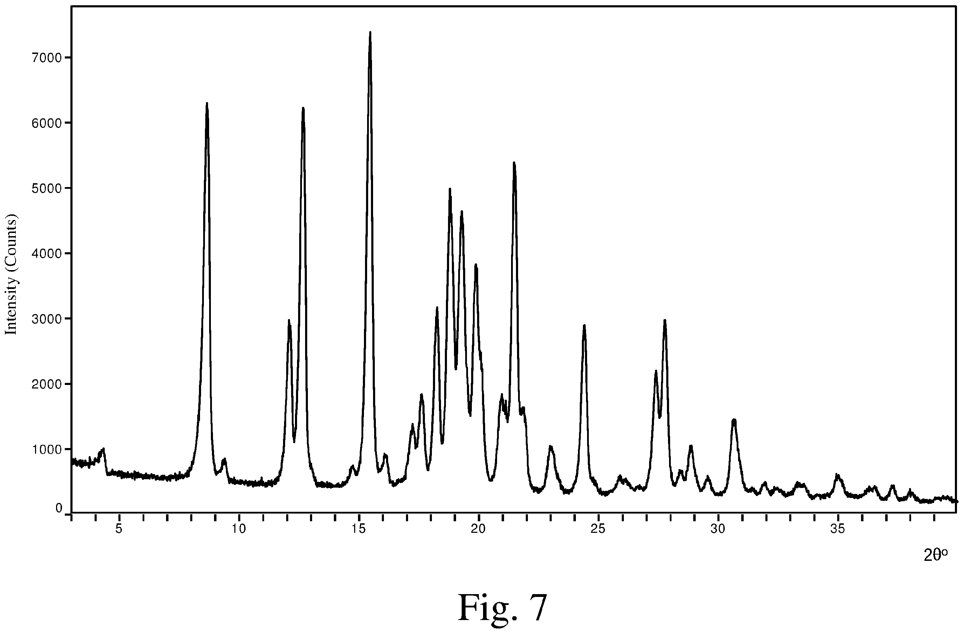

In a more preferred embodiment, the crystalline form IV of the compound of Formula (1) has an XRPD pattern comprising peaks at diffraction angles (2Θ) essentially the same as shown in Figure 7. In the most preferred embodiment, the XRPD pattern of the crystalline form IV of the compound of Formula (1) is essentially the same as shown in Figure 7, and preferably is as shown in Figure 7.

In a preferred embodiment, the crystalline form IV of the compound of Formula (1) of the present invention has a DSC graph comprising characteristic peaks at about 169 ± 2°C and 222 ± 2°C (the onset temperature).

In a preferred embodiment, the crystalline form IV of the compound of Formula (1) has almost no weight loss when heated to about 200°C.

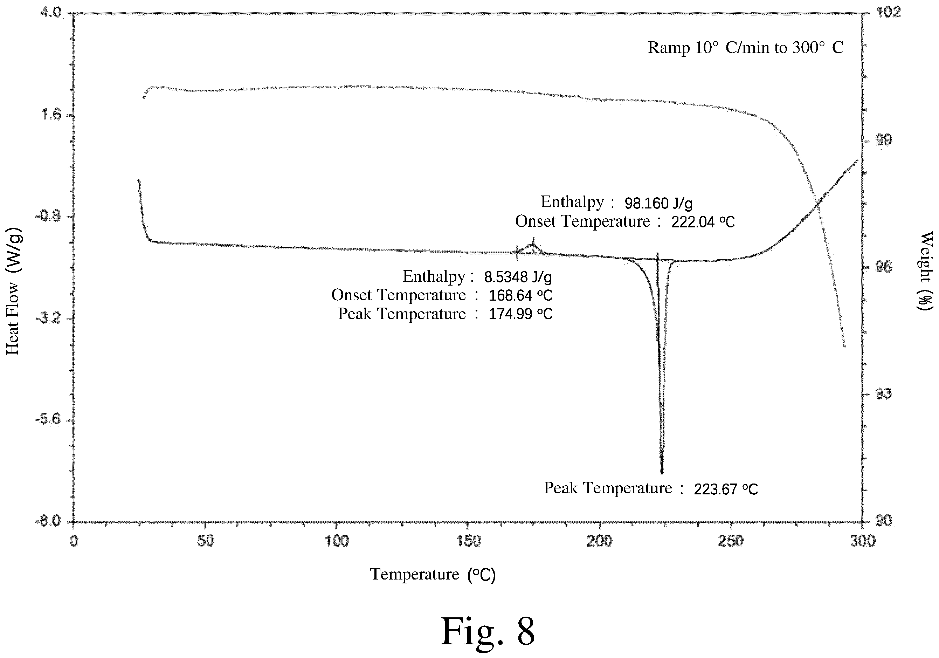

In a more preferred embodiment, the crystalline form IV of the compound of Formula (1) has a DSC-TGA graph comprising characteristic peaks essentially the same as shown in Figure 8. In the most preferred embodiment, the DSC-TGA graph of the crystalline form IV of the compound of Formula (1) is essentially the same as shown in Figure 8, and preferably is as shown in Figure 8.

In a preferred embodiment, the crystalline form IV of the compound of Formula (1) is an anhydrous crystalline form.

In an embodiment, the present invention provides crystalline form V of the compound of Formula (1), wherein the crystalline form V has an XRPD pattern comprising characteristic peaks at diffraction angles (2Θ) of about 5.9±0.2°, 8.3±0.2°, 11.9±0.2°, 13.4±0.2°, 16.8±0.2°, 17.6±0.2°, 18.5±0.2°, 20.7±0.2°, 24.0±0.2° and 28.2±0.2°.

In a preferred embodiment, the crystalline form V of the compound of Formula (1) has an XRPD pattern comprising peaks at the following diffraction angles (2θ):

| 2θ (°) ± 0.2° | Intensity% ± 5% |
|---|---|
| 5.9 | 11 |
| 8.3 | 43 |
| 11.9 | 46 |
| 13.4 | 11 |
| 16.8 | 100 |
| 17.6 | 15 |
| 18.5 | 41 |
| 20.7 | 31 |
| 24.0 | 18 |
| 28.2 | 14 |

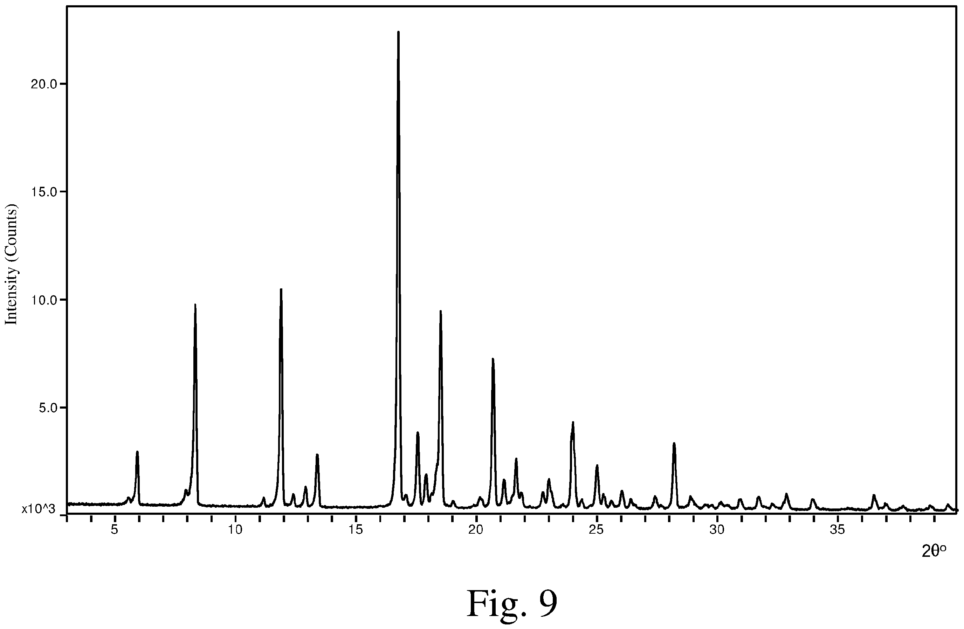

In a more preferred embodiment, the crystalline form V of the compound of Formula (1) has an XRPD pattern comprising peaks at diffraction angles (2Θ) essentially the same as shown in Figure 9. In the most preferred embodiment, the XRPD pattern of the crystalline form V of the compound of Formula (1) is essentially the same as shown in Figure 9, and preferably is as shown in Figure 9.

In a preferred embodiment, the crystalline form V of the compound of Formula (1) has a DSC graph comprising characteristic peaks at about 102 ± 2°C, 113 ± 2°C and 224 ± 2°C (the onset temperature).

In a preferred embodiment, the crystalline form V of the compound of Formula (1) of the present invention has a weight loss of about 15% when heated to about 200°C.

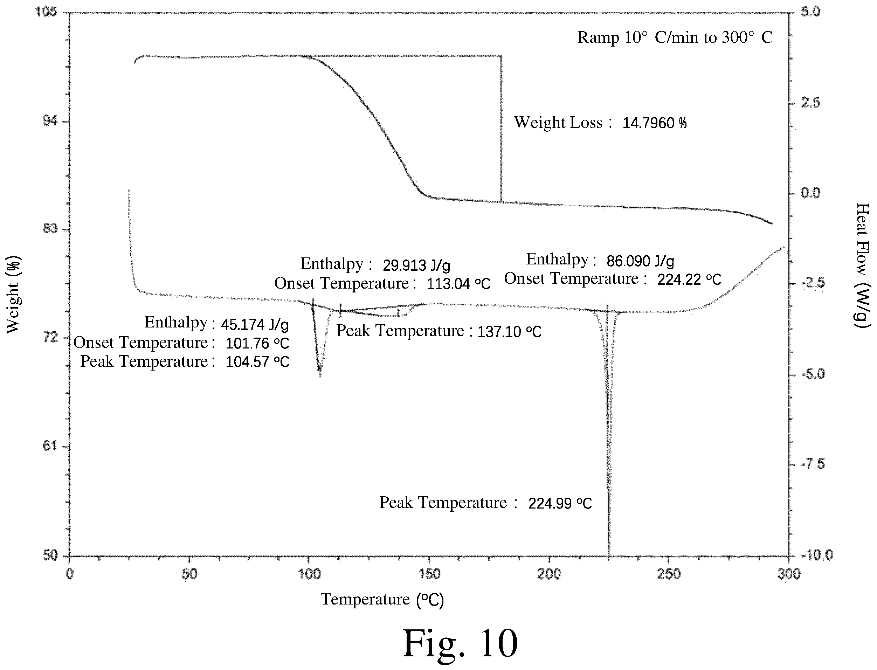

In a more preferred embodiment, the crystalline form V of the compound of Formula (1) has a DSC-TGA graph comprising characteristic peaks essentially the same as shown in Figure 10. In the most preferred embodiment, the DSC-TGA graph of the crystalline form V of the compound of Formula (1) is essentially the same as shown in Figure 10, and preferably is as shown in Figure 10.

In a preferred embodiment, the crystalline form V of the compound of Formula (1) is a solvate with dimethyl sulfoxide, wherein the molar ratio of the compound of Formula (1) to dimethyl sulfoxide preferably is 1:1.

In some embodiments, the present invention further provides a method of preparing any one of the crystalline forms I-V, and the method includes, but is not limited to, a room temperature solvent volatilization method, a suspension stirring method, an anti-solvent addition method and a cooling method, *etc.*

In some embodiments, the crystalline form is prepared by a room temperature solvent volatilization method, which comprises completely dissolving a solid of the compound of Formula (1) in a solvent to form a clear solution (the solution may be filtered as needed to provide a clear solution), and then allowing the resulting solution to stand at room temperature, so that the solvent completely volatilizes, to afford the crystalline form.

In some embodiments, the solvent includes but is not limited to organic solvents, such as alcohols, hydrocarbons (including alkanes, halogenated alkanes, alkenes, alkynes, and aromatic hydrocarbons), ethers (including chain ethers and cyclic ethers (such as furans (including tetrahydrofurans) and dioxanes)), ketones, nitriles, or esters having 1-10 carbon atoms, specifically such as methanol, ethanol, isopropanol, dichloromethane, trichloromethane (chloroform), tetrahydrofuran, acetone, butanone, methyl tert-butyl ether, ethyl acetate, or acetonitrile, or a mixed solvent formed by two or more of the above solvents.

In some embodiments, when the crystalline form I is prepared using the room temperature solvent volatilization method, the solvent used includes but is not limited to organic solvents, such as hydrocarbons (including alkanes, halogenated alkanes, alkenes, alkynes, and aromatic hydrocarbons), ethers (including chain ethers and cyclic ethers (such as furans (including tetrahydrofurans) and dioxanes)) or ketones having 1-10 carbon atoms, specifically such as dichloromethane, tetrahydrofuran, acetone, or butanone, or a mixed solvent formed by two or more of the above solvents.

In some embodiments, when the crystalline form II is prepared using the room temperature solvent volatilization method, the solvent used includes but is not limited to organic solvents, such as alcohols or nitriles having 1-10 carbon atoms, specifically ethanol or acetonitrile, or a mixed solvent formed by two or more of the above solvents.

In some embodiments, the weight to volume ratio (mg/mL) of the compound of Formula (1) to the solvent is (5-20):1, preferably about 10:1.

In some embodiments, the crystalline form is prepared by a suspension stirring method, which comprises adding a solid of the compound of Formula (1) to a solvent to obtain a suspension, stirring, and then separating to afford the crystalline form.

In some embodiments, the stirring is performed at room temperature or at an elevated temperature (for example, 40-60°C, preferably about 50°C).

In some embodiments, the solvent includes but is not limited to inorganic solvents (such as water) and organic solvents (such as alcohols, ketones, hydrocarbons (including alkanes, halogenated alkanes, alkenes, alkynes, and aromatic hydrocarbons), ethers (including chain ethers and cyclic ethers (such as furans (including tetrahydrofurans) and dioxanes)), esters, nitriles, and organic acids having 1-10 carbon atoms, such as methanol, n-propanol, isopropanol, acetone, butanone, methyl isobutyl ketone, methyl acetate, ethyl acetate, isopropyl acetate, butyl acetate, acetonitrile, hexane, heptane, dichloromethane, methyl tert-butyl ether, dioxane, dimethyl carbonate, tetrahydrofuran, 2-methyltetrahydrofuran, acetic acid, toluene, trichloromethane, cyclopentyl methyl ether), or a mixed solvent of two or more selected from the above solvents.

In some embodiments, the preferred mixed solvent is as shown in the table below:

| **Solvent₁** | **Solvent₂** |
|---|---|
| methanol | water |
| acetone | water |
| acetonitrile | water |
| methanol | ethyl acetate |
| methanol | methyl *tert*-butyl ether |
| methanol | acetonitrile |
| ethanol | butyl acetate |
| ethanol | n-heptane |
| acetone | isopropanol |
| acetone | ethyl acetate |
| acetonitrile | methyl acetate |
| acetone | n-heptane |
| acetone | acetonitrile |
| butanone | n-heptane |

In some embodiments, the volume ratio of solvent₁ to solvent₂ is from 1:1 to 1:5, preferably from 1:1 to 1:3.

In some embodiments, the weight to volume ratio (mg/mL) of the compound of Formula (1) to the solvent is from (20-350):1, preferably (20-300):1, more preferably (60-300):1, and most preferably (60-150):1.

In some embodiments, the crystalline form is prepared by an anti-solvent addition method, which comprises dissolving a solid of the compound of Formula (1) in a good solvent to form a clear solution (the solution may be filtered as needed to provide a clear solution), then adding an anti-solvent thereto to allow the precipitation of the solid, which is filtered to afford the crystalline form.

In some embodiments, the good solvent includes but is not limited to organic solvents such as alcohols, ketones, hydrocarbons (including alkanes, halogenated alkanes, alkenes, alkynes, and aromatic hydrocarbons), ethers (including chain ethers and cyclic ethers (such as furans (including tetrahydrofurans) and dioxanes)), sulfones, amides, and organic acids having 1 to 10 carbon atoms, such as methanol, ethanol, acetone, tetrahydrofuran, acetic acid, trichloromethane, dimethyl sulfoxide, or dimethylacetamide. In some embodiments, the anti-solvent includes but is not limited to inorganic solvents (such as water) and organic solvents (such as ketones, hydrocarbons (including alkanes, halogenated alkanes, alkenes, alkynes, and aromatic hydrocarbons), ethers (including chain ethers and cyclic ethers (such as furans (including tetrahydrofurans) and dioxanes)), esters, and nitriles having 1 to 10 carbon atoms), such as n-hexane, n-heptane, cyclopentyl methyl ether, acetonitrile, methyl isobutyl ketone, 2-methyltetrahydrofuran, dioxane, isopropyl acetate, dichloromethane, toluene, acetonitrile, butanone, methyl tert-butyl ether, ethyl isopropionate, dimethyl carbonate, and ethyl acetate.

In some embodiments, when the crystalline form I is prepared using the anti-solvent addition method, the good solvent employed is ketones having 1-10 carbon atoms (preferably acetone); and the anti-solvent employed is an inorganic solvent (preferably water) or hydrocarbons having 1-10 carbon atoms (preferably n-heptane).

In some embodiments, when the crystalline form III is prepared using the anti-solvent addition method, the good solvent employed is ethers having 1-10 carbon atoms (preferably tetrahydrofuran); and the anti-solvent employed is hydrocarbons having 1-10 carbon atoms (preferably n-heptane).

In some embodiments, when the crystalline form IV is prepared using the anti-solvent addition method, the good solvent employed is alcohols (preferably methanol) or sulfones (preferably dimethyl sulfoxide) having 1-10 carbon atoms; and the anti-solvent employed is an inorganic solvent (preferably water).

In some embodiments, the volume ratio of the good solvent to the anti-solvent is from (0.5-1):(1-20), preferably about 1:10. In some embodiments, the weight to volume ratio (mg/mL) of the compound of Formula (1) to the good solvent is from (50-200):1, preferably from (90-150):1.

In some embodiments, the crystalline form is prepared by a cooling method, which comprises adding a solid of the compound of Formula (1) to a solvent, heating and stirring to dissolve the solid, allowing the resulting clear solution (the solution may be filtered as needed to provide a clear solution) to stand, and slowly cooling to afford the crystalline form.

In some embodiments, the solvent includes but is not limited to inorganic solvents (such as water) and organic solvents, such as alcohols, ketones, hydrocarbons (including alkanes, halogenated alkanes, alkenes, alkynes, and aromatic hydrocarbons), ethers (including chain ethers and cyclic ethers (such as furans (including tetrahydrofurans) and dioxanes)), nitriles, esters, and sulfones having 1 to 10 carbon atoms, specifically such as isopropanol, acetone, butanone, trichloromethane, acetonitrile, tetrahydrofuran, methanol, n-hexane, cyclohexane, methyl acetate, ethyl acetate, or dimethyl sulfoxide, or a mixed solvent of two or more selected from the above solvents.

In some embodiments, the preferred mixed solvent is as shown in the table below:

| **Solvent₁** | **Solvent₂** |
|---|---|
| acetone | water |
| tetrahydrofuran | cyclohexane |
| dimethyl sulfoxide | water |

In some embodiments, the volume ratio of solvent 1 to solvent 2 is from 5:1 to 1:5, preferably about 1:1.

In some embodiments, when the crystalline form I is prepared using the cooling method, the solvent employed is alcohols having 1-10 carbon atoms, preferably isopropanol.

In some embodiments, when the crystalline form II is prepared using the cooling method, the solvent employed is nitriles (preferably acetonitrile), ketones (preferably butanone), esters (preferably methyl acetate) having 1-10 carbon atoms, or a mixed solvent of water and a ketone solvent having 1-10 carbon atoms (preferably acetone).

In some embodiments, when the crystalline form III is prepared using the cooling method, the solvent employed is a mixed solvent of tetrahydrofuran and a hydrocarbon solvent having 1-10 carbon atoms (preferably cyclohexane).

In some embodiments, when the crystalline form V is prepared using the cooling method, the solvent employed is a mixed solvent of water and dimethyl sulfoxide.

In some embodiments, the weight to volume ratio (mg/mL) of the compound of Formula (1) to the solvent is from (60-150):1.

### Pharmaceutical composition and use

In another embodiment, the present application provides a pharmaceutical composition comprising any one or more of the crystalline forms I, II, III, IV or V of the compound of Formula (1) of the present invention, and one or more pharmaceutically acceptable carriers.

In another embodiment, the present application provides use of the crystalline form I, II, III, IV or V of the compound of Formula (1) of the present invention in the manufacture of a medicament for treating acute or chronic pain, migraine, osteoarthritis, rheumatoid arthritis, gout, bursitis, ankylosing spondylitis, primary dysmenorrhea, cancer or arteriosclerosis.

In another embodiment, the present application provides the crystalline form I, II, III, IV or V of the compound of Formula (1) of the present invention for use in treating acute or chronic pain, migraine, osteoarthritis, rheumatoid arthritis, gout, bursitis, ankylosing spondylitis, primary dysmenorrhea, cancer or arteriosclerosis.

In another embodiment, the present application provides a method for treating acute or chronic pain, migraine, osteoarthritis, rheumatoid arthritis, gout, bursitis, ankylosing spondylitis, primary dysmenorrhea, cancer or arteriosclerosis, comprising administering to a subject in need thereof, preferably a mammal, a prophylactically or therapeutically effective amount of any one or more of the crystalline forms I, II, III, IV or V of the compound of Formula (1) of the present invention.

In another embodiment, the present application provides use of the crystalline form I, II, III, IV or V of the compound of Formula (1) of the present invention in the manufacture of a medicament for treating cancer.

In another embodiment, the present application provides the crystalline form I, II, III, IV or V of the compound of Formula (1) of the present invention for use in treating cancer.

In another embodiment, the present application provides a method for treating cancer, comprising administering to a subject in need thereof, preferably a mammal, a prophylactically or therapeutically effective amount of any one or more of crystalline forms I, II, III, IV or V of the compound of Formula (1) of the present invention.

In a preferred embodiment, the cancer is selected from the group consisting of breast cancer, cervical cancer, colorectal cancer, endometrial cancer, glioblastoma, head and neck cancer, kidney cancer, liver cancer, lung cancer, medulloblastoma, ovarian cancer, pancreatic cancer, prostate cancer, skin cancer and urethral cancer.

As used herein, the term "pharmaceutically acceptable carrier" in the present invention refers to a diluent, auxiliary material, excipient, or vehicle with which a therapeutic is administered, and it is, within the scope of sound medical judgment, suitable for contact with the tissues of human beings and animals without excessive toxicity, irritation, allergic response, or other problem or complication, commensurate with a reasonable benefit/risk ratio.

The pharmaceutically acceptable carrier which can be employed in the pharmaceutical composition of the present invention includes, but is not limited to sterile liquids, such as water and oils, including those of petroleum, animal, vegetable or synthetic origin, such as peanut oil, soybean oil, mineral oil, sesame oil and the like. Water is an exemplary carrier when the pharmaceutical composition is administered intravenously. Physiological salines as well as aqueous dextrose and glycerol solutions can also be employed as liquid carriers, particularly for injectable solutions. Suitable pharmaceutical excipients include starch, glucose, lactose, sucrose, gelatin, maltose, chalk, silica gel, sodium stearate, glycerol monostearate, talc, sodium chloride, dried skim milk, glycerol, propylene glycol, water, ethanol and the like. The composition, if desired, can also contain minor amounts of wetting or emulsifying agents, or pH buffering agents. Oral formulations can include standard carriers such as pharmaceutical grades of mannitol, lactose, starch, magnesium stearate, sodium saccharine, cellulose, magnesium carbonate, *etc.* Examples of suitable pharmaceutical carriers are described in e.g. Remington's Pharmaceutical Sciences (1990).

The composition of the present invention can act systemically and/or topically. To this end, it can be administered through a suitable route, such as through injection, intravenous, intraarterial, subcutaneous, intraperitoneal, intramuscular, or transdermal administration, or administered via oral, buccal, nasal, transmucosal, topical, as an ophthalmic formulation, or via inhalation.

For these routes of administration, the composition of the present invention can be administered in a suitable dosage form.

The dosage form may be solid, semi-solid, liquid, or gas formulations, specifically including, but not limited to, tablets, capsules, powders, granules, lozenges, hard candies, powders, sprays, creams, salves, suppositories, gels, pastes, lotions, ointments, aqueous suspensions, injectable solutions, suspensions, elixirs, and syrups.

The pharmaceutical composition of the present invention may be manufactured by any process well known in the art, e.g., by means of mixing, dissolving, granulating, dragee-making, levigating, emulsifying, lyophilizing processes, or the like.

As used herein, the term "therapeutically effective amount" refers to the amount of a compound being administered which will relieve to some extent one or more of the symptoms of the disorder being treated.

Dosage regimens may be adjusted to provide the optimum desired response. For example, a single bolus may be administered, several divided doses may be administered over time, or the dose may be proportionally reduced or increased as indicated by the exigencies of the therapeutic situation. It is to be noted that dosage values may vary with the type and severity of the condition to be alleviated, and may include single or multiple doses. It is to be further understood that for any particular subject, specific dosage regimens should be adjusted over time according to the individual need and the professional judgment of the person administering or supervising the administration of the composition.

The amount of the compound of the present invention administered will be dependent on the subject being treated, the severity of the disorder or condition, the rate of administration, the disposition of the compound and the discretion of the prescribing physician. Generally, an effective dosage is in the range of about 0.0001 to about 50 mg per kg body weight per day, for example about 0.01 to about 10 mg/kg/day, in single or divided doses. For a 70 kg human, this would amount to about 0.007 mg to about 3500 mg/day, for example about 0.7 mg to about 700 mg/day. In some instances, dosage levels below the lower limit of the aforesaid range may be more than adequate, while in other cases, still larger doses may be employed without causing any harmful side effect, provided that such larger doses are first divided into several small doses for administration throughout the day.

The content or dosage of the compound of the present invention in the pharmaceutical composition is about 0.01 mg to about 1000 mg, suitably 0.1-500 mg, preferably 0.5-300 mg, more preferably 1-150 mg, particularly preferably 1-50 mg, e.g., 1.5 mg, 2 mg, 4 mg, 10 mg, and 25 mg, *etc.*

Unless otherwise indicated, the term "treating" or "treatment", as used herein, means reversing, alleviating, inhibiting the progress of, or preventing a disorder, condition, or disease to which such term applies, or one or more symptoms of such disorder, condition, or disease.

As used herein, the term "subject" includes a human or non-human animal. An exemplary human subject includes a human subject having a disease (such as one described herein) (referred to as a patient), or a normal subject. The term "non-human animal" as used herein includes all vertebrates, such as non-mammals (e.g., birds, amphibians, reptiles) and mammals, such as non-human primates, livestock and/or domesticated animals (such as sheep, dog, cat, cow, pig and the like).

### Examples

The present invention is explained in more detail below with reference to the examples, which are only used to illustrate the technical solutions of the present invention, and are not intended to limit the scope thereof, and those skilled in the art may make some non-essential improvements and adjustments, which still fall within the scope of the present invention.

The model and parameters of the equipment used in the examples and experimental examples are as follows:

### 1. X-ray Powder Diffraction (XRPD)

The solid samples obtained in the examples were analyzed for crystalline form using a PANalytical EMPYREAN equipped with a PIXceI^{1D} detector. The instrument parameters are as follows: scanning range: 3° (2θ) to 40° (2θ); step size: 0.013° (2θ); tube voltage and current are 45 KV and 40 mA, respectively.

### 2. Thermogravimetric Analysis (TGA)

Thermogravimetric analysis of the samples was performed using a TGA 55 (TA Instruments, US). The samples were placed in an open aluminum sample pan, automatically weighed inside the TGA furnace, and then heated to the final temperature at a rate of 10°C/min.

### 3. Differential Scanning Calorimetry (DSC)

Thermal analysis of the samples was conducted using a Discovery DSC 250 (TA Instruments, US). Approximately 2 mg of the sample was weighed and placed in a DSC sample pan. The sample was equilibrated at 25°C and then heated to the final temperature at a rate of 10°C/min.

### 4. High-Performance Liquid Chromatography Analysis (HPLC)

The HPLC measurements were performed using an Agilent HPLC 1260 series instrument. The HPLC measurement method parameters are as listed in the table below.

### Parameters of the HPLC Method

| | |
|---|---|
| **Chromatographic column** | Chromatographic column: SunFire C18 4.6*150mm*3.5um |
| **Mobile Phase** | A: water with 0.02% trifluoroacetic acid |
| | B: acetonitrile with 0.02% trifluoroacetic acid |
| **Time/Components (T/B%)** | 0/10, 8/90, 13/90, 13.1/10 |
| **Column Temperature** | 30°C |
| **Detector** | DAD; 250 nm |
| **Flow Rate** | 1.0 mL/min |
| **Injection Volume** | 5 µL |
| **Run Time** | 13.1 min |
| **Post-Run Time** | 5 min |
| **Diluent** | methanol |

### Example 1

### Preparation of the compound of Formula (1) (3-(1-(1-(benzofuran-2-ylmethyl)-1H-indole-7-carboxamido)cyclopropyl)bicyclo[1.1.1]pentane-1-carboxyl ic acid)

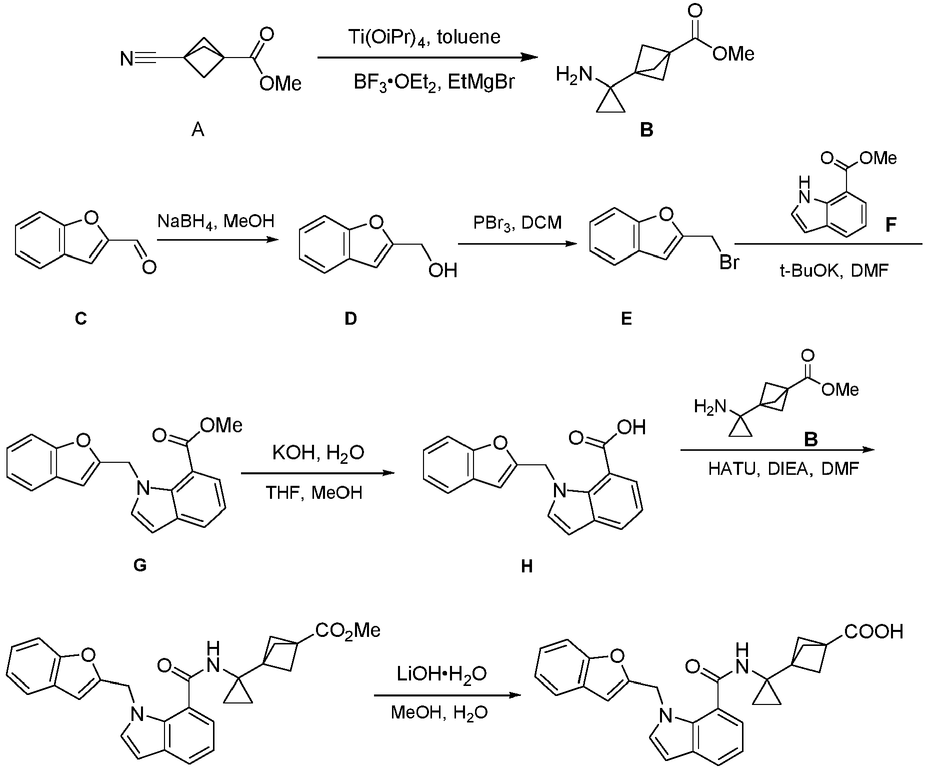

### Step 1: synthesis of methyl 3-(1-aminocyclopropyl)bicyclo[1.1.1]pentane-1-carboxylate B

Titanium tetraisopropoxide (29.8 g, 99.7 mmol, 31 mL, purity: 95%) was added to a solution of methyl 3-cyanobicyclo[1.1.1]pentane-1-carboxylate (**A**) (22.5 g, 99.2 mmol) in toluene (240 mL) under a nitrogen atmosphere at -20°C. EtMgBr (3 M, 60 mL) was added dropwise within 30 minutes under a nitrogen atmosphere at -20°C, and the temperature was kept between -20 ~ -10°C. After stirring for 30 minutes, BF₃·Et₂O (27.6 g, 194 mmol, 24 mL) was added dropwise. The reaction mixture was stirred at -20°C for 30 minutes and then at 25°C for 12 hours. The reaction mixture was quenched by slowly adding aqueous hydrochloric acid (1 N, 30 mL) at 0°C, and then the separated organic layer was discarded. The aqueous phase was basified to pH ~ 12 with a 10 M aqueous sodium hydroxide solution at 0°C, and extracted with ethyl acetate (200 mL × 2). The combined organic layer was concentrated, and the resulting residue was purified by flash column chromatography on silica gel to obtain compound **B** (3.9 g, 21.7 mmol, yield: 21.9%) as a yellow solid. MS (ESI): 182.3 [M+1]⁺.

¹H NMR (400 MHz, DMSO-*d₆*) δ: 3.58 (s, 3H), 1.77 (s, 6H), 0.39-0.37 (m, 4H).

### Step 2: synthesis of benzofuran-2-ylmethanol D

Benzofuran-2-carbaldehyde (**C**) (3 g, 20.5 mmol) and anhydrous methanol (40 ml) were added successively to a reaction flask, and the resulting mixture was cool to 0°C. Sodium borohydride (0.545 g, 14.4 mmol) was added thereto in batches, and the temperature was kept below 25°C. After completion of the addition, the reaction mixture was stirred at room temperature for 1 hour. After completion of the reaction, the solvent was removed under reduced pressure. 1 N aqueous HCl solution (15 ml) was added, and the resulting mixture was stirred at room temperature for 5 minutes. The mixture was adjusted to pH 8-9 with saturated aqueous sodium bicarbonate solution, and extracted with ethyl acetate (10 ml × 3). The combined organic phase was washed with saturated brine, dried over anhydrous sodium sulfate, and concentrated under reduced pressure to remove the solvent, to obtain compound **D** (3.0 g, 20.27 mmol, yield: 98.9%) as a yellow oil. MS (ESI): 149.1 [M+1]⁺.

¹H NMR (400 MHz, CDCl₃) δ: 7.55 (dd, *J* = 8.4, 7.2 Hz, 1H), 7.47 (dd, *J* = 8.8, 7.6 Hz, 1H), 7.29-7.19 (m, 2H), 6.66 (s, 1H), 4.77 (d, *J* = 4.8 Hz, 2H).

### Step 3: synthesis of 2-(bromomethyl)benzofuran E

Compound **D** (2.47 g, 16.7 mmol) and dry dichloromethane (32 ml) were added successively to a reaction flask, and the resulting mixture was cooled to 0°C. Phosphorus tribromide (1.72 mL, 18.4 mmol) was slowly added dropwise thereto. After completion of the addition, the reaction mixture was warmed up to room temperature and stirred for 1 hour. TLC showed that the reaction was completed. The reaction mixture was adjusted to pH 8-9 with saturated aqueous sodium bicarbonate solution, and extracted with dichloromethane (10 ml × 3). The combined organic phase was washed with saturated brine, dried over anhydrous sodium sulfate, and concentrated under reduced pressure to remove the solvent, to obtain compound E (3.36 g, yield: 95.9%) as a yellow oil. The compound was directly used in the next reaction without further purification.

### Step 4: synthesis of methyl 1-(benzofuran-2-ylmethyl)-1H-indole-7-carboxylate G

At 0 °C, potassium *tert*-butoxide (1.53 g, 13.63 mmol) was added to a solution of methyl 1H-indole-7-carboxylate **F** (1.59 g, 9.09 mmol) in DMF (80 mL), followed by the addition of 2-(bromomethyl)benzofuran **E** (2.5 g, 11.36 mmol). The resulting mixture was then warmed to 25 °C and stirred for 3 hours. After completion of the reaction was confirmed by TLC (petroleum ether: ethyl acetate = 9:1), the reaction mixture was poured into 200 mL of water and extracted with ethyl acetate (100 mL × 2). The organic layer was washed with brine (200 mL), dried over anhydrous sodium sulfate, filtered, and concentrated. The resulting residue was purified by flash chromatography on silica gel to afford compound **G** (2.0 g, 6.56 mmol, yield: 72.2%) as a yellow oil. MS (ESI): 306.2 [M+1]⁺.

### Step 5: synthesis of 1-(benzofuran-2-ylmethyl)-1H-indole-7-carboxylic acid H

To a solution of compound **G** (2.0 g, 6.56 mmol) in methanol (40 mL) and tetrahydrofuran (40 mL), an aqueous solution of KOH (2M, 33 mL) was added, and the resulting mixture was heated to 50 °C and stirred for 12 hours. After the starting material was completely consumed and the target product was detected by LCMS, the reaction mixture was concentrated at 45 °C to remove most of the methanol and tetrahydrofuran. The mixture was acidified to pH ~6-7 with a 1N hydrochloric acid aqueous solution, washed with 1N hydrochloric acid, and extracted with ethyl acetate (60 mL × 3). The organic layer was dried over anhydrous sodium sulfate, filtered, and concentrated to afford compound **H** (1.8 g, 6.19 mmol, yield: 94.3%) as a light yellow solid. MS (ESI): 292.1 [M+1]⁺.

### Step 6: synthesis of methyl 3-(1-(1-(benzofuran-2-ylmethyl)-1H-indole-7-carboxamido)cyclopropyl)bicyclo[1.1.1]pentane-1-carboxyla te J

DIEA (2.8 g, 21.6 mmol) was added to a solution of compound **H** (1.8 g, 6.18 mmol), compound **B** (1.4 g, 7.73 mmol) and HATU (3.08 g, 8.12 mmol) in DMF (40 mL). The resulting mixture was stirred at 25 °C under nitrogen for 3 hours. After LCMS indicated that the reaction was complete, the reaction mixture was poured into 100 mL of water and extracted with ethyl acetate (50 mL × 3). The organic layer was washed with brine (50 mL × 2), dried over anhydrous sodium sulfate, filtered, and concentrated under reduced pressure. The residue was purified by flash column chromatography on silica gel to afford compound **J** (2.3 g, 5.07 mmol, yield: 82.0%) as a pale yellow solid. MS (ESI): 455.0 [M+1]⁺.

### Step 7: synthesis of 3-(1-(1-(benzofuran-2-ylmethyl)-1H-indole-7-carboxamido)cyclopropyl)bicyclo[1.1.1]pentane-1-carboxyli c acid 1

LiOH·H₂O (2 M, 3.3 mL, 6.6 mmol) was added to a solution of compound **J** (2.3 g, 5.07 mmol) in methanol (50 mL). The resulting mixture was stirred at 50 °C for 24 hours. After LCMS indicated that the reaction was complete, the reaction mixture was concentrated at 50 °C to remove most of the methanol. Water (30 mL) was added, and the mixture was acidified to pH ~5 with 1N hydrochloric acid and extracted with ethyl acetate (40 mL × 3). The combined organic layers were washed with brine (50 mL × 2), dried over anhydrous sodium sulfate, filtered, and concentrated. The residue was washed with ethyl ether (20 mL), and freeze-dried, to afford the compound of Formula (1) (1.8 g, 4.09 mmol, yield: 80.7%) as an off-white powder. MS (ESI): 441.0 [M+1]⁺.

¹H NMR (400 MHz, DMSO-*d₆*) δ: 12.24 (s, 1H), 8.75 (s, 1H), 7.69 (d, J = 7.6 Hz, 1H), 7.51 (d, J = 3.2 Hz, 1H), 7.45 (t, J = 7.8 Hz, 2H), 7.24-7.06 (m, 4H), 6.60 (d, J = 3.2 Hz, 1H), 6.18 (s, 1H), 5.76 (s, 2H), 1.76 (s, 6H), 0.63 (d, J = 7.2 Hz, 2H), 0.50 (t, J = 5.6 Hz, 2H).

### Example 2: Room Temperature Solvent Volatilization Method

A solid of the compound of Formula (1) obtained in Example 1 was completely dissolved in the following solvent (the concentration of the solution is about 10 mg/mL). The resulting solution was then allowed to stand at room temperature to allow the solvent to completely volatilize, resulting in a solid. XRPD analysis was performed on the obtained solid, and the crystalline form was determined from the resulting XRPD patterns (when the XRPD pattern was essentially the same as shown in Figure 1, then the crystalline form was determined as form I; when the XRPD pattern was essentially the same as shown in Figure 3, then it was determined as form II; when the XRPD pattern was essentially the same as shown in Figure 5, then it was determined as form III; when the XRPD pattern was essentially the same as shown in Figure 7, then it was determined as form IV; and when the XRPD pattern was essentially the same as shown in Figure 9, then it was determined as form V The same standards were applied in Examples 3-9). The results are shown in the table below.

| **Solvent** | **Crystalline form** |
|---|---|
| tetrahydrofuran | Crystalline form I |
| acetone | Crystalline form I |
| butanone | Crystalline form I |
| dichloromethane | Crystalline form I |
| acetonitrile | Crystalline form II |
| ethanol | Crystalline form II |

### Example 3: Room Temperature Suspension Stirring Method in Single Solvent

A solid of the compound of Formula (1) obtained from Example 1, weighing 30 milligrams, was added to the following solvent at the specified volume to form a suspension. The suspension was then stirred at room temperature for 3 days, followed by filtration to obtain a solid. XRPD analysis was carried out on the resulting solid, and the crystalline form was determined from the XRPD patterns. The results are shown in the table below.

| **Solvent** | **Volume (mL)** | **Crystalline form** |
|---|---|---|
| methanol | 0.1 | Crystalline form II |
| isopropanol | 0.5 | Crystalline form II |
| acetone | 0.1 | Crystalline form II |
| butanone | 0.5 | Crystalline form II |
| methyl acetate | 0.5 | Crystalline form II |
| ethyl acetate | 0.5 | Crystalline form II |
| butyl acetate | 0.5 | Crystalline form II |
| isopropyl acetate | 0.5 | Crystalline form II |
| acetonitrile | 0.5 | Crystalline form II |

### Example 4: Room Temperature Suspension Stirring Method in Mixed Solvent

A solid of the compound of Formula (1) obtained from Example 1, weighing 30 milligrams, was placed in each of the mixed solvents as shown in the table below to form a suspension. The suspension was then stirred at room temperature for 3 days, followed by filtration to obtain a solid. XRPD analysis was carried out on the resulting solid, and the crystalline form was determined from the XRPD patterns. The results are shown in the table below.

| **Solvent₁** | **Solvent₂** | **Volume₁/Volume₂** | **Total volume of the solvent (mL)** | **Crystalline form** |
|---|---|---|---|---|
| methanol | water | 1/1 | 0.5 | Crystalline form II |
| acetone | water | 1/1 | 0.5 | Crystalline form II |
| acetonitrile | water | 1/1 | 0.5 | Crystalline form II |
| methanol | ethyl acetate | 1/3 | 0.5 | Crystalline form II |
| methanol | methyl *tert*-butyl ether | 1/3 | 0.5 | Crystalline form II |
| methanol | acetonitrile | 1/3 | 0.5 | Crystalline form II |
| ethanol | butyl acetate | 1/3 | 0.5 | Crystalline form II |
| ethanol | n-heptane | 1/1 | 0.5 | Crystalline form II |
| acetone | isopropanol | 1/3 | 0.5 | Crystalline form II |
| acetone | ethyl acetate | 1/1 | 0.5 | Crystalline form II |
| acetonitrile | methyl acetate | 1/1 | 0.5 | Crystalline form II |
| acetone | n-heptane | 1/1 | 0.5 | Crystalline form II |
| acetone | acetonitrile | 1/1 | 0.5 | Crystalline form II |
| butanone | n-heptane | 1/1 | 0.5 | Crystalline form II |

### Example 5: High-Temperature Suspension Stirring Method in Single Solvent

A solid of the compound of Formula (1) obtained from Example 1, weighing 30 milligrams, was added to the following solvent at the specified volume to form a suspension. The suspension was then stirred at 50°C for 3 days, followed by filtration to obtain a solid. XRPD analysis was carried out on the resulting solid, and the crystalline form was determined from the XRPD patterns. The results are shown in the table below.

| **Solvent** | **Volume (mL)** | **Crystalline form** |
|---|---|---|
| methanol | 0.1 | Crystalline form II |
| isopropanol | 0.2 | Crystalline form II |
| butanone | 0.2 | Crystalline form II |
| acetonitrile | 0.2 | Crystalline form II |
| dichloromethane | 0.2 | Crystalline form II |
| methyl acetate | 0.2 | Crystalline form II |
| ethyl acetate | 0.2 | Crystalline form II |
| isopropyl acetate | 0.2 | Crystalline form II |

### Example 6: High-Temperature Suspension Stirring Method in Mixed Solvent

A solid of the compound of Formula (1) obtained from Example 1, weighing 30 milligrams, was placed in each of the mixed solvents as shown in the table below to form a suspension. The suspension was then stirred at 50°C for 3 days, followed by filtration to obtain a solid. XRPD analysis was carried out on the resulting solid, and the crystalline form was determined from the XRPD patterns. The results are shown in the table below.

| **Solvent₁** | **Solvent₂** | **Volume₁/Volume₂** | **Total volume of the solvent (mL)** | **Crystalline form** |
|---|---|---|---|---|
| methanol | ethyl acetate | 1/3 | 0.5 | Crystalline form II |
| acetone | ethyl acetate | 1/1 | 0.5 | Crystalline form II |
| acetone | isopropanol | 1/3 | 0.5 | Crystalline form II |
| acetonitrile | methyl acetate | 1/1 | 0.5 | Crystalline form II |
| acetone | acetonitrile | 1/3 | 0.5 | Crystalline form II |

### Example 7: Anti-solvent Addition Method

As shown in the table below, a specified amount of the solid of the compound of Formula (1) obtained from Example 1 was weighted and placed into a good solvent, the resulting solution was filtered, and the filtrate was slowly added to an anti-solvent. The precipitated solid was filtered and subjected to XRPD analysis. The crystalline form was determined from the XRPD patterns, and the results are shown in the table below.

| **The amount of the compound of Formula (1)** | **Good solvent** | **Anti-solvent** | **Volume of the good solvent/volume of the anti-solvent** | **Crystalline form** |
|---|---|---|---|---|
| 150 mg | acetone | water | 1 mL / 10 mL | Crystalline form I |
| | | n-heptane | 1 mL / 10 mL | Crystalline form I |
| 90 mg | tetrahydrofuran | n-heptane | 1 mL / 10 mL | Crystalline form III |
| 90 mg | dimethyl sulfoxide | water | 1 mL / 10 mL | Crystalline form IV |
| 150 mg | methanol | water | 1 mL / 10 mL | Crystalline form IV |

### Example 8: Cooling Method in Single Solvent

Approximately 30 mg of the solid of the compound of Formula (1) obtained from Example 1 was weighed and added to each of the solvents as shown in the table below, and the resulting suspension was heated to completely dissolve the solid. The solution was then cooled. The precipitated solid was filtered and analyzed by XRPD. The crystalline form was determined from the XRPD patterns, and the results are shown in the table below.

| **Solvent (0.5 mL)** | **Crystalline form** |
|---|---|
| isopropanol | Crystalline form I |
| acetonitrile | Crystalline form II |
| butanone | Crystalline form II |
| methyl acetate | Crystalline form II |

### Example 9: Cooling Method in Mixed Solvent

Approximately 30 mg of the solid compound of Formula (1) obtained from Example 1 was weighed and added to each of the mixed solvents as shown in the table below, and the resulting suspension was heated to completely dissolve the solid. The solution was filtered, and the filtrate was cooled to room temperature. The precipitated solid was filtered and analyzed by XRPD. The crystalline form was determined from the XRPD patterns, and the results are shown in the table below.

| **Solvent₁** | **Solvent₂** | **Volume₁/Volume₂** | **Total volume of the solvent (mL)** | **Crystalline form** |
|---|---|---|---|---|
| acetone | water | 1/1 | 0.2 mL | Crystalline form II |
| tetrahydrofuran | cyclohexane | 1/1 | 0.2 mL | Crystalline form III |
| dimethyl sulfoxide | water | 1/1 | 0.2 mL | Crystalline form V |

### Example 10: Thermal Gravimetric Analysis and Differential Scanning Calorimetry of Crystalline forms

Thermal gravimetric analysis and differential scanning calorimetry were performed on crystalline forms I, II, III, IV, and V The DSC and TGA graphs for crystalline forms I, II, III, IV, and V are shown in Figures 2, 4, 6, 8, and 10, respectively.

### Experimental Examples

### Experimental Example 1: Mechanical Grinding Test

0.5 g of crystalline form II was placed in a mortar and ground for about 5 minutes, then the solid was collected for XRPD testing.

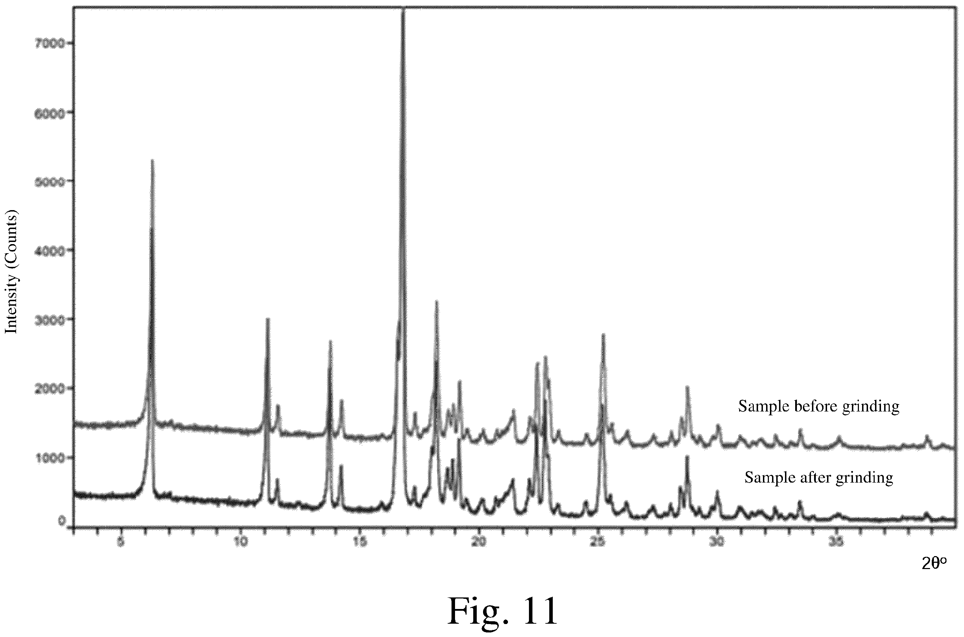

The XRPD patterns of the starting sample and the sample after grinding are shown in Figure 11. The results indicated that the crystalline form of crystalline form II remained unchanged after grinding.

### Experimental Example 2: Crystalline Form Transformation Test

Crystalline form IV was slowly heated to 200°C and then cooled to room temperature. Samples before and after heating were collected for XRPD testing.

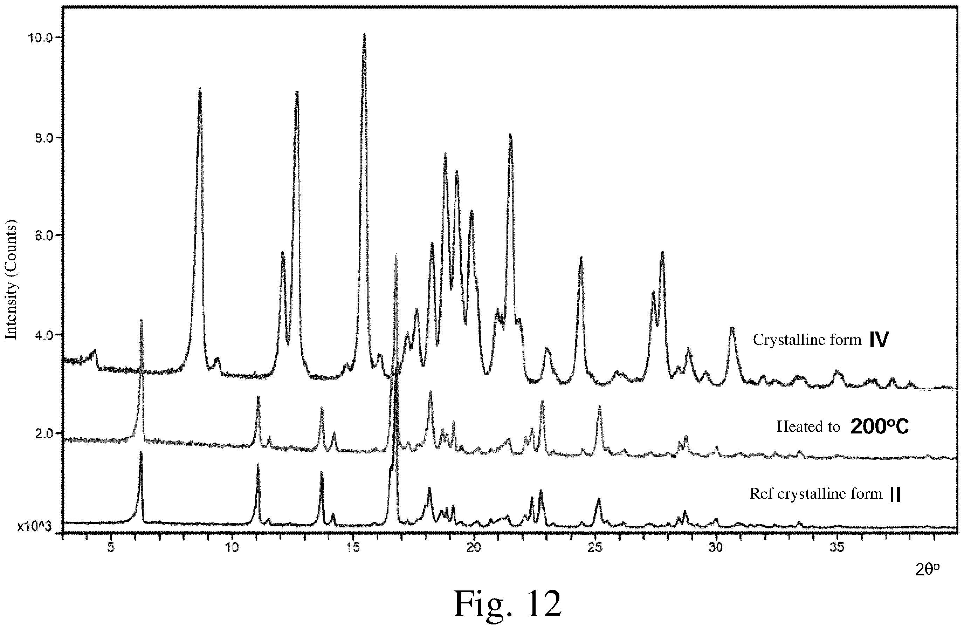

The XRPD patterns before and after heating, compared with the reference crystalline form II, are shown in Figure 12. The results indicated that crystalline form IV transformed into crystalline form II upon heating. Therefore, crystalline form II exhibited better stability compared to crystalline form IV

### Experimental Example 3: Solid Stability Test

Crystalline form II was stored separately at a sealed condition at 60°C and at 40°C/75% RH for 7 days. The purity of the samples was determined by HPLC before and after storage, and the XRPD patterns were measured.

The purity determination results of the samples are shown in the table below. The results indicated that after storage at a sealed condition at 60°C and at 40°C/75% RH, the purity of the samples did not decrease.

| **Sample** | **Purity at the beginning (Area%)** | **Purity - Day 7 (Area%)** | |
|---|---|---|---|
| | | **40°C/75%RH** | **60°C** |
| Crystalline form II | 99.4 | 99.4 | 99.6 |

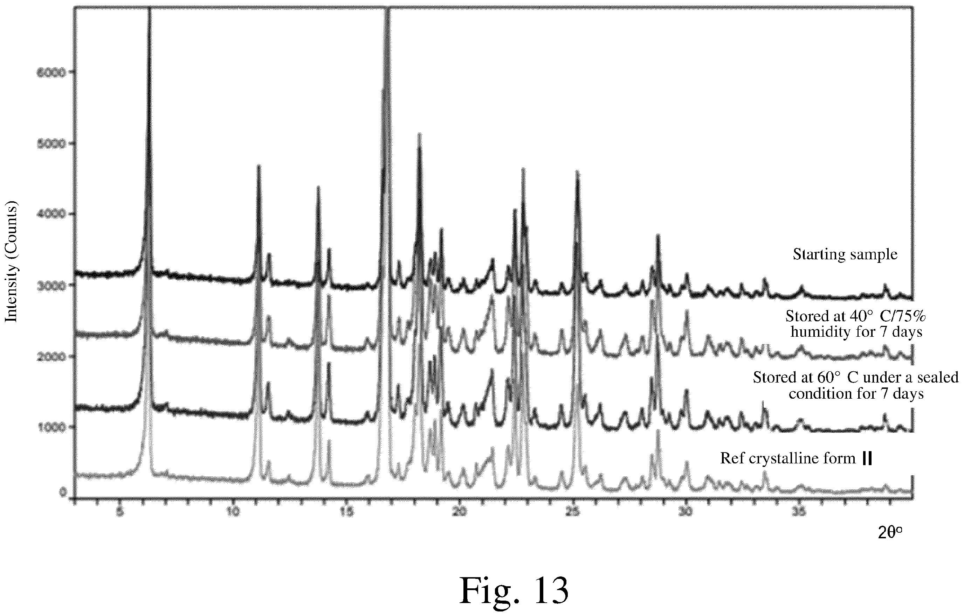

The XRPD patterns of the samples before and after storage are shown in Figure 13. The results indicated that the crystalline form of crystalline form II remained unchanged after being placed at a sealed condition at 60°C and at 40°C/75% RH for 7 days.

### Experimental Example 4: Light Exposure Test

Crystalline form II of the compound of Formula (1) was placed under conditions of light intensity not less than 1.2×10⁶ Lux·hr, with near-ultraviolet energy not less than 200 w·hr/m², at 25°C, RH 25% for 30 days. Samples were taken at 0, 5, 10, and 30 days, respectively, to observe changes in sample characteristics, test for drying loss, measure the total impurity content by HPLC, and measure the XRPD patterns of the samples.

The test results showed that after the sample was placed under light exposure conditions (total illumination not less than 1.2×10⁶ Lux·hr, near-ultraviolet energy not less than 200 w·hr/m²) for 30 days, there were no significant changes in moisture and content compared to the results on day 0, and no new impurities greater than 0.05% were formed. XRPD patterns indicated that the sample's crystalline form did not change.

The results indicated that crystalline form II was stable under light exposure conditions (total illumination not less than 1.2×10⁶ Lux·hr, near-ultraviolet energy not less than 200 w·hr/m²).

### Experimental Example 5: High Temperature Test

Crystalline form II of the compound of Formula (1) was placed at a high temperature of 60°C for 30 days. Samples were taken at 0, 5, 10, and 30 days, respectively, to observe changes in sample characteristics, measure specific rotation with a polarimeter, test for drying loss, measure the total impurity content by HPLC, and measure the XRPD patterns of the samples.

The test results showed that after being placed at a high temperature of 60°C for 30 days, there were no significant differences in appearance, moisture, content, and crystalline form compared to the results on day 0, and no new impurities greater than 0.05% were formed.

The results indicated that the sample was stable under high-temperature (60°C) conditions.

### Experimental Example 6: High Humidity Test

Crystalline form II of the compound of Formula (1) was placed at 25°C and 92.5% high humidity for 30 days. Samples were taken at 0, 5, 10, and 30 days, respectively, to observe changes in sample characteristics, test for drying loss, measure the total impurity content by HPLC, and measure the XRPD patterns of the samples.

The test results showed that after being placed under high humidity conditions of 92.5% RH for 30 days, the water absorption was 0.03% (less than 5%); there were no significant differences in appearance, moisture, content, and crystalline form compared to the results on day 0, and no new impurities greater than 0.05% were formed.

The results indicated that the sample was stable under high humidity (RH 92.5%) conditions.

### Experimental Example 7: Pharmacokinetic Test in SD Rats

A clear solution of crystalline form II of the compound of Formula (1) was prepared in 10% DMSO, 60% PEG 400, and 30% water for injection and administered intravenously to SD rats. An aqueous suspension of crystalline form II of the compound of Formula (1) in 0.5% sodium carboxymethylcellulose (CMC.Na) was prepared and administered orally to SD rats to investigate its pharmacokinetic characteristics.

After a single intravenous administration of 15 mg/kg of the compound of Formula (1) to SD rats, the drug exposure (AUC₀₋ₜ) of the compound in SD rats was 78,811 ng*hr/mL, with a clearance (CL) and steady-state apparent volume of distribution (Vss) of 3.59 mL/min/kg and 1.18 L/kg, respectively.

After a single oral administration of 40 mg/kg of the compound of Formula (1) to SD rats, the maximum blood drug concentration (Cmax) and exposure (AUCo-t) of the compound were 27,700 ng/mL and 153,279 ng*hr/mL, respectively.

It is apparent that crystalline form II of the compound of Formula (1) has excellent blood drug concentration and exposure.

### Experimental Example 8: Pharmacokinetic Test in Beagle Dogs

A clear solution of crystalline form II of the compound of Formula (1) was prepared in 10% DMSO, 60% PEG 400, and 30% water for injection and administered intravenously to beagle dogs. An aqueous suspension of crystalline form II of the compound of Formula (1) in 0.5% sodium carboxymethylcellulose (CMC.Na) was prepared and administered orally to beagle dogs to investigate its pharmacokinetic characteristics.

After a single intravenous administration of 5 mg/kg of the compound of Formula (1) to beagle dogs, the drug exposure (AUCo-t) of the compound in beagle dogs was 12,649 ng*hr/mL, with a clearance (CL) and steady-state apparent volume of distribution (Vss) of 6.64 mL/min/kg and 1.50 L/kg, indicating that the compound of Formula (1) is a compound with low clearance and is widely distributed in the body.

After a single oral administration of 45 mg/kg of the compound of Formula (1) to beagle dogs, the maximum blood drug concentration (Cmax) and exposure (AUCo-t) of the compound were 32,917 ng/mL and 79,576 ng*hr/mL, respectively.

It is apparent that crystalline form II of the compound of Formula (1) has excellent blood drug concentration and exposure.

The above specific embodiments describe the present invention in further detail. However, the scope of the above-mentioned subject matter of the present invention should not be construed as being limited to the above examples, and the technical solutions implemented based on the disclosure of the present invention are all within the scope of the present invention.

## Claims

1. Crystalline form I of the compound of Formula (1): 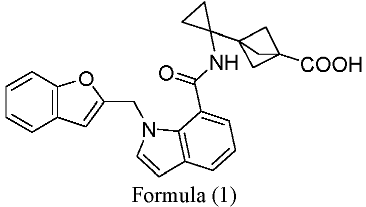 wherein the crystalline form I has an XRPD pattern comprising characteristic peaks at diffraction angles (2Θ) of about 6.3±0.2°, 11.0±0.2°, 13.5±0.2°, 16.7±0.2°, 18.3±0.2°, 18.6±0.2°, 19.0±0.2°, 22.1±0.2°, 22.8±0.2° and 25.3±0.2°.

2. A method of preparing the crystalline form I of the compound of Formula (1) according to claim 1, wherein the method is selected from the group consisting of a room temperature solvent volatilization method, an anti-solvent addition method and a cooling method.

3. Crystalline form II of the compound of Formula (1): 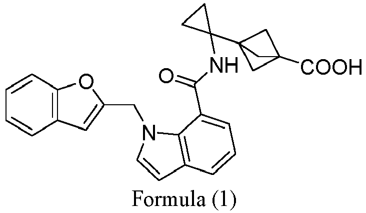 wherein the crystalline form II has an XRPD pattern comprising characteristic peaks at diffraction angles (2θ) of 6.3±0.2°, 11.1±0.2°, 13.8±0.2°, 16.6±0.2°, 16.8±0.2°, 18.2±0.2°, 19.2±0.2°, 22.4±0.2°, 22.8±0.2° and 25.2±0.2°.

4. A method of preparing the crystalline form II of the compound of Formula (1) according to claim 3, wherein the method is selected from the group consisting of a room temperature solvent volatilization method, a suspension stirring method and a cooling method.

5. Crystalline form III of the compound of Formula (1): 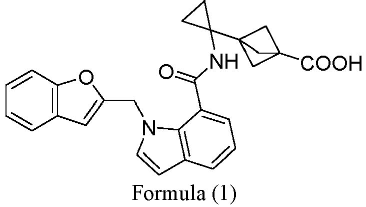
wherein the crystalline form III has an XRPD pattern comprising characteristic peaks at diffraction angles (2θ) of 5.3±0.2°, 10.7±0.2°, 12.0±0.2°, 17.7±0.2°, 18.1±0.2°, 21.6±0.2°, 22.1±0.2°, 26.9±0.2°, 31.9±0.2° and 32.6±0.2°;
the crystalline form III preferably is a solvate with tetrahydrofuran, wherein the molar ratio of the compound of Formula (1) to tetrahydrofuran preferably is 1:1.

6. A method of preparing the crystalline form III of the compound of Formula (1) according to claim 5, wherein the method is selected from the group consisting of an anti-solvent addition method and a cooling method.

7. Crystalline form IV of the compound of Formula (1): 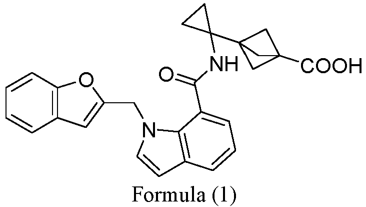 wherein the crystalline form IV has an XRPD pattern comprising characteristic peaks at diffraction angles (2θ) of 8.7±0.2°, 12.1±0.2°, 12.7±0.2°, 15.5±0.2°, 18.3±0.2°, 18.8±0.2°, 19.3±0.2°, 19.9±0.2°, 21.5±0.2°, 24.4±0.2° and 27.8±0.2°.

8. A method of preparing the crystalline form IV of the compound of Formula (1) according to claim 7, wherein the method is an anti-solvent addition method, comprising dissolving a solid of the compound of Formula (1) in a good solvent to form a clear solution (the solution may be filtered as needed to provide a clear solution), then adding an anti-solvent thereto to allow the precipitation of the solid, which is filtered to afford the crystalline form IV;
the good solvent employed preferably is alcohols (preferably methanol) or sulfones (preferably dimethyl sulfoxide) having 1 to 10 carbon atoms, and the anti-solvent employed preferably is an inorganic solvent (preferably water).

9. Crystalline form V of the compound of Formula (1): 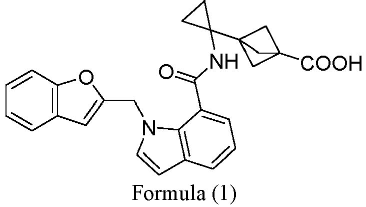
wherein the crystalline form V has an XRPD pattern comprising characteristic peaks at diffraction angles (2θ) of 5.9±0.2°, 8.3±0.2°, 11.9±0.2°, 13.4±0.2°, 16.8±0.2°, 17.6±0.2°, 18.5±0.2°, 20.7±0.2°, 24.0±0.2° and 28.2±0.2°;
the crystalline form V preferably is a solvate with dimethyl sulfoxide, wherein the molar ratio of the compound of Formula (1) to dimethyl sulfoxide preferably is 1:1.

10. A method of preparing the crystalline form V of the compound of Formula (1) according to claim 9, wherein the method is a cooling method, comprising adding a solid of the compound of Formula (1) to a solvent, heating and stirring to dissolve the solid, allowing the resulting clear solution (the solution may be filtered as needed to provide a clear solution) to stand, and slowly cooling to afford the crystalline form V;
the solvent employed preferably is a mixed solvent of water and dimethyl sulfoxide.

11. A pharmaceutical composition comprising the crystalline form I, II, III, IV or V of the compound of Formula (1) according to any one of claims 1, 3, 5, 7 and 9, and one or more pharmaceutically acceptable carriers.

12. Use of the crystalline form I, II, III, IV or V of the compound of Formula (1) according to any one of claims 1, 3, 5, 7 and 9 in the manufacture of a medicament for treating acute or chronic pain, migraine, osteoarthritis, rheumatoid arthritis, gout, bursitis, ankylosing spondylitis, primary dysmenorrhea, cancer or arteriosclerosis.
